# FASCICULE DE BREVET EUROPEEN

(11) **EP 0 631 777 B1**
(45) Date de publication et mention de la délivrance du brevet: **22.11.2000**
(21) Numéro de dépôt: 94401461.2
(22) Date de dépôt: 28.06.1994
(51) Int. Cl.: A61K 31/175, A61K 31/165, A61K 31/12

(54) **Utilisation de dérivés de la bêta-naphtoquinone pour l'inhibition de l'agrégation plaquettaire**
Verwendung von Beta-Naphthochinonderivaten zur Hemmung von Blutplättchen-Aggregation
Use of derivatives of beta-naphthoquinone for inhibiting platelet aggregation

(30) Priorité: 02.07.1993 FR 9308112
(43) Date de publication de la demande: 04.01.1995
(73) Titulaire: HOECHST MARION ROUSSEL, 92800 Puteaux (FR)
(72) Inventeur: Blache, Denis, F-21800 Chevigny Saint Sauveur (FR); Bloy, Christian, F-75015 Paris (FR); Hercelin, Bernard, F-60600 Clermont (FR)
(74) Mandataire: Vieillefosse, Jean-Claude

(56) Documents cités:
- FR-M- 924
- LA GAZETTE MEDICALE, vol.100, no.20, 27 Mai 1993 page 38 PLOIN, M. 'ETIOVEN 10mg, M DICAMENT DE L'ANN E'
- AGRESSOLOGIE, vol.12, no.1, 1971 pages 25 - 30 LABORIT, H. ET AL 'EFFETS DE LA MONO-SEMICARBAZONE DE LA BETA-NAPHTOQUINONE DANS LE CHOC H MORRAGIQUE EXP RIMENTAL'

## Description

La présente invention concerne une nouvelle utilisation de dérivés de la béta-naphtoquinone ainsi que de leurs sels.

Le brevet Spécial de Médicament 924 M décrit l'application à titre de médicaments de dérivés de la béta-naphtoquinone. Selon ce brevet, ces dérivés sont présentés comme possédant des propriétés hémostatiques et des propriétés vitaminiques.

On sait que les plaquettes sanguines interagissent avec les facteurs de la coagulation et les composants de la paroi vasculaire pour jouer un rôle primordial d'une part favorable, dans le maintien de l'intégrité vasculaire, dans l'hémostase et, d'autre part défavorable, dans l'initiation des thromboses et dans le développement de l'athérosclérose.

En poursuivant ses études sur les dérivés de la béta-naphtoquinone précités, la demanderesse vient de trouver, de façon tout à fait inattendue, que ces derniers dérivés présentaient une remarquable activité inhibitrice de l'agrégation plaquettaire.

La présente demande a ainsi pour objet une nouvelle utilisation de dérivés de la béta-naphtoquinone de formule générale (I) : 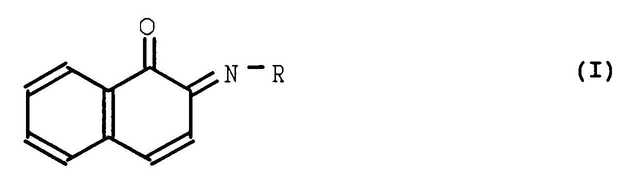 dans laquelle R représente un groupement de formule -NH-CO-NH₂, ou un groupement -NH-CO-CH₃ ou un groupement hydroxyle, ainsi que leurs sels d'addition avec les acides minéraux ou organiques pharmaceutiquement acceptables, pour la fabrication d'un médicament permettant d'inhiber l'agrégation plaquettaire.

Les sels d'addition avec les acides minéraux ou organiques pharmaceutiquement acceptables peuvent être, par exemple, les sels formés avec les acides chlorhydrique, bromhydrique, iodhydrique, nitrique, sulfurique, phosphorique, propionique, formique, benzoïque, maléïque, fumarique, succinique, tartrique, citrique, oxalique, glyoxylique, aspartique, alcanesulfoniques tels que les acides méthane et éthane sulfoniques et arylsulfoniques, tels que l'acide benzènesulfonique.

Parmi les dérivés de la béta-naphtoquinone de formule générale (I), on retient notamment :
- le produit de formule (I) dans laquelle R représente un groupement de formule -NH-CO-NH₂, ainsi que ses sels d'addition avec les acides minéraux ou organiques pharmaceutiquement acceptables,
- le produit de formule (I) dans laquelle R représente un groupement de formule -NH-CO-CH₃, ainsi que ses sels d'addition avec les acides minéraux ou organiques pharmaceutiquement acceptables,
- le produit de formule (I) dans laquelle R représente un groupement hydroxy, ainsi que ses sels d'addition avec les acides minéraux ou organiques pharmaceutiquement acceptables.

Parmi les produits préférés de l'invention, utilisés pour la fabrication d'un médicament permettant d'inhiber l'agrégation plaquettaire, on retient tout particulièrement la 2-semicarbazone de la 1,2-naphtoquinone plus connue sous sa dénomination commune internationale de naftazone. Dans la partie expérimentale, ce produit figure sous sa DCI (dénomination commune internationale).

En raison de leurs remarquables propriétés inhibitrices de l'agrégation plaquettaire, illustrées plus loin dans la partie expérimentale, les dérivés de la béta-naphtoquinone de formule (I), tels que définis ci-dessus, ainsi que leurs sels d'addition avec les acides minéraux ou organiques pharmaceutiquement acceptables, peuvent être utilisés dans le traitement et la prévention des accidents ischémiques, dans le traitement des troubles plaquettaires.

La dose usuelle, variable selon le produit utilisé, le sujet traité et l'affection en cause peut être par exemple de 1 mg à 100 mg par jour, par voie orale ou par voie injectable.

L'invention a également pour objet les compositions pharmaceutiques qui renferment à titre de principe actif l'un au moins des médicaments définis ci-dessus.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les capsules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les divers agents mouillants, dispersants ou émulsifiants, les conservateurs.

Des procédés de préparation des dérivés de la béta-naphtoquinone de formule (I) ainsi que de leurs sels ont été décrits dans la littérature. On peut notamment citer les procédés décrits dans le brevet spécial de médicament 924M précité, ou dans le brevet français 2 103 504.

Il va être donné maintenant à titre non limitatif des exemples de compositions pharmaceutiques pouvant être utilisées dans la mise oeuvre de l'invention.

### EXEMPLE 1 :

On a préparé des comprimés répondant à la formule suivante :

| | |
|---|---|
| - Naftazone | 10 mg |
| - Excipient q.s.p. pour un comprimé terminé à | 150 mg |

(détail de l'excipient : lactose, amidon, talc, stéarate de magnésium).

### EXEMPLE 2 : :

On a préparé un soluté injectable répondant à la formule suivante :

| | |
|---|---|
| - Naftazone | 5 mg |
| - Excipient aqueux stérile q.s.p. | 2 ml |

### EXEMPLE 3 :

On a introduit 8,6 g de Naftazone dans 200 ml d'anhydride acétique. Après 10 heures de chauffage à 140°C, puis refroidissement, filtration, extraction du précipité par le chloroforme, évaporation à sec, reprise par l'éthanol, passage sur charbon actif, filtration et cristallisation lente, on a obtenu 5,1 g de produit répondant à la formule (I) avec R = -NH-CO-CH₃ (poudre microcristalline jaune ocre, F = 137-138°C).

### ETUDE PHARMACOLOGIQUE

### I - Inhibition de l'agrégation plaquettaire

A l'heure actuelle, de nombreux composés sont capables d'inhiber les fonctions plaquettaires mais seulement deux, l'aspirine et la ticlopidine, ont une activité pharmacologique reconnue : l'inhibition de l'agrégation plaquettaire ex vivo.

Nous avons donc testé de la naftazone en comparaison de la ticlopidine et de l'aspirine ex vivo chez le rat et, dans un deuxième temps, in vitro sur des plaquettes de rat et humaines.

### In vitro :

L'agrégation des plaquettes humaines ou de rat isolées de leur plasma et stimulées par l'ADP ou la thrombine en présence de différentes concentrations de naftazone (10⁻⁴ M à 10⁻⁸ M) est diminuée de façon importante et significative. Elle est environ, de l'ordre de 70 à 20 % pour les concentrations de naftazone allant de 10⁻⁴ M à 10⁻⁶ M, respectivement.

### Ex vivo :

L'effet comparé de la naftazone avec l'aspirine et la ticlopidine chez le rat a permis de montrer, après injections répétées de 10 mg/kg/jour de chacun des produits, une inhibition de l'agrégation plaquettaire induite à l'ADP ou à la thrombine importante et significative (tableau 1).

| Groupe | (n) | Plasma riche en plaquettes | | Plaquettes lavées | |
|---|---|---|---|---|---|
| | | Thrombine | ADP | Thrombine | ADP |
| D Naftazone | (6) | 32.9±7.7^{b} | 20.6±5.6^{c,d} | 24.3±18.8^{a,b,c,d} | 20.7±19.2a,b,c,d |

Les résultats de l'agrégation induite par la thrombine ou l'ADP sont exprimés (moyenne ± D.S) en pourcentage de transmission optique. Les nombres d'une même colonne affectés de la même lettre sont significativement différents (au minimum, p < 0.05) suivant le test de Newman-Keuls après analyse de la variance.

Ces résultats montrent donc que, dans les conditions expérimentales utilisées, la naftazone inhibe de manière signficative l'agrégation plaquettaire induite à l'ADP ou à la thrombine. Cette inhibition, mise en évidence in vitro sur les plaquettes humaines ou de rat, a été confirmée ex vivo chez le rat. De plus, cet effet est équivalent à celui mis en évidence avec la ticlopidine et l'aspirine dans les mêmes conditions.

## Revendications

1. Utilisation de dérivés de la béta-naphtoquinone de formule générale (I) : 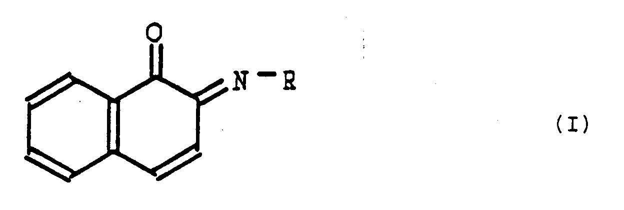 dans laquelle R représente un groupement de formule -NH-CO-NH₂, ou un groupement de formule -NH-CO-CH₃ou un groupement hydroxyle, ainsi que leurs sels d'addition avec les acides minéraux ou organiques pharmaceutiquement acceptables, pour la fabrication d'un médicament permettant d'inhiber l'agrégation plaquettaire pour le traitement ou la prévention des accidents ischémiques, dans le traitement des trouble plaquettaires.

2. Utilisation selon la revendication 1, caractérisée en ce que le dérivé de la béta-naphtoquinone est un produit de formule générale (I) dans laquelle R représente un groupement de formule -NH-CO-NH₂, ainsi que ses sels d'addition avec les acides minéraux ou organiques pharmaceutiquement acceptables.

3. Utilisation selon la revendication 1, caractérisée en ce que le dérivé de la béta-naphtoquinone est un produit de formule générale (I) dans laquelle R représente un groupement de formule -NH-CO-CH₃, ainsi que ses sels d'addition avec les acides minéraux ou organiques pharmaceutiquement acceptables.

4. Utilisation selon la revendication 1, caractérisée en ce que le dérivé de la béta-naphtoquinone est un produit de formule générale (I) dans laquelle R représente un groupement hydroxy, ainsi que ses sels d'addition avec les acides minéraux ou organiques pharmaceutiquement acceptables.

5. Utilisation selon la revendication 1 ou 2, caractérisée en ce que le dérivé de la béta-naphtoquinone est la 2-semicarbazone de la 1,2-naphtoquinone.

## Claims

1. Use of derivatives of beta-naphthoquinone of general formula (I): 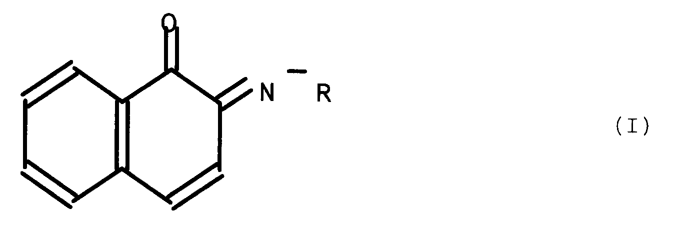 in which R represents a group of formula -NH-CO-NH₂, or a group of formula -NH-CO-CH₃ or a hydroxyl group, as well as their addition salts with pharmaceutically acceptable mineral or organic acids, for the production of a medicament allowing the inhibition of platelet aggregation for the treatment or the prevention of ischemic complications, in the treatment of platelet disorders.

2. Use according to claim 1, characterized in that the derivative of beta-naphthoquinone is a product of general formula (I) in which R represents a group of formula -NH-CO-NH₂, as well as its addition salts with pharmaceutically acceptable mineral or organic acids.

3. Use according to claim 1, characterized in that the derivative of beta-naphthoquinone is a product of general formula (I) in which R represents a group of formula -NH-CO-CH₃, as well as its addition salts with pharmaceutically acceptable mineral or organic acids.

4. Use according to claim 1, characterized in that the derivative of beta-naphthoquinone is a product of general formula (I) in which R represents a hydroxy group, as well as its addition salts with pharmaceutically acceptable mineral or organic acids.

5. Use according to claim 1 or 2, characterized in that the derivative of beta-naphthoquinone is 1,2-naphtoquinone 2-semicarbazone.

## Patentansprüche

1. Verwendung von beta-Naphthochinon-Derivaten der allgemeinen Formel (I): 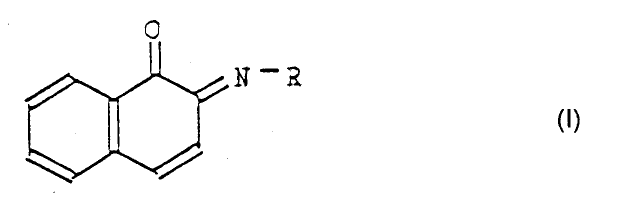 in der R eine Gruppe der Formel -NH-CO-NH₂ oder eine Gruppe der Formel -NH-CO-CH₃ oder eine Hydroxygruppe darstellt, sowie deren Additionssalzen mit den pharmazeutisch annehmbaren mineralischen oder organischen Säuren zur Herstellung eines Medikaments, das es ermöglicht, die Blutplättchenaggregation zu hemmen, zur Behandlung oder Vorbeugung der ischämischen Anfälle bei der Behandlung der Blutplättchenstörungen.

2. Verwendung gemäß Anspruch 1, dadurch gekennzeichnet, dass das beta-Naphthochinon-Derivat ein Produkt der allgemeinen Formel (I) ist, in der R eine Gruppe der Formel -NH-CO-NH₂ darstellt, sowie seiner Additionssalze mit den pharmazeutisch annehmbaren mineralischen oder organischen Säuren.

3. Verwendung gemäß Anspruch 1, dadurch gekennzeichnet, dass das beta-Naphthochinon-Derivat ein Produkt der allgemeinen Formel (I) ist, in der R eine Gruppe der Formel -NH-CO-CH₃ darstellt, sowie seiner Additionssalze mit den pharmazeutisch annehmbaren mineralischen oder organischen Säuren.

4. Verwendung gemäß Anspruch 1, dadurch gekennzeichnet, dass das beta-Naphthochinon-Derivat ein Produkt der allgemeinen Formel (I) ist, in der R eine Hydroxygruppe darstellt, sowie seiner Additionssalze mit den pharmazeutisch annehmbaren mineralischen oder organischen Säuren.

5. Verwendung gemäß Anspruch 1 oder 2, dadurch gekennzeichnet, dass das beta-Naphthochinon-Derivat 1,2-Naphthochinon-2-semicarbazon ist.
